(19) Europäisches Patentamt
European Patent Office
Office européen des brevets

(11) **EP 4 268 809 A1**

(12) **DEMANDE DE BREVET EUROPEEN**

(43) Date de publication:
**01.11.2023 Bulletin 2023/44**

(21) Numéro de dépôt: **23170542.7**

(22) Date de dépôt: **28.04.2023**

(51) Classification Internationale des Brevets (IPC):
*A61K 31/01* (2006.01)     *A61K 9/00* (2006.01)
*A61K 45/06* (2006.01)     *A61P 33/14* (2006.01)
*A01N 27/00* (2006.01)     *A01P 7/04* (2006.01)
*A61Q 17/02* (2006.01)

(52) Classification Coopérative des Brevets (CPC):
**A61K 31/01; A01N 27/00; A01P 7/04;
A61K 9/0014; A61K 45/06; A61P 33/14;
A61Q 17/02**

(84) Etats contractants désignés:
**AL AT BE BG CH CY CZ DE DK EE ES FI FR GB
GR HR HU IE IS IT LI LT LU LV MC ME MK MT NL
NO PL PT RO RS SE SI SK SM TR**
Etats d'extension désignés:
**BA**
Etats de validation désignés:
**KH MA MD TN**

(30) Priorité: **28.04.2022 FR 2203979**

(71) Demandeurs:
• **Pagnotta Grislain, Nadine**
  **33200 Bordeaux (FR)**

• **Grislain, Luc**
  **33200 Bordeaux (FR)**

(72) Inventeurs:
• **Pagnotta Grislain, Nadine**
  **33200 Bordeaux (FR)**
• **Grislain, Luc**
  **33200 Bordeaux (FR)**

(74) Mandataire: **Aquinov
  12, Cours Xavier Arnozan
  33000 Bordeaux (FR)**

(54) **COMPOSITION HUILEUSE ANTI-POUX À BASE D'ALCANES LIQUIDES ET SES UTILISATIONS**

(57)     La présente invention concerne une nouvelle composition huileuse à base d'alcanes liquides pour prévenir et/ou traiter des pédiculoses, en particulier des pédiculoses de la tête chez l'homme ou sur le pelage animal.

**EP 4 268 809 A1**

**Description**

**Domaine technique**

L'invention se rapporte au domaine de la lutte contre les poux et les lentes de tête. En particulier, l'invention concerne une nouvelle composition huileuse à base d'alcanes liquides, et ses utilisations.

**Etat de l'art**

Les poux sont des insectes hématophages pouvant être responsables de maladies parasitaires fréquentes et contagieuses appelées pédiculoses ou phitiriases. Trois types de poux en particulier sont responsables de ces ectoparasitoses :

- *Pediculus humanus corporis,* le pou de corps, vivant dans les coutures et les plis de vêtements, ne passant sur la peau que pour se nourrir, responsable de pédiculoses corporelles,
- *Phtirius pubis,* le pou du pubis et des autres régions du corps couvertes de poils grossiers ou morpion, responsable de phitiriases pubiennes, et
- *Pediculus humanus capitis :* le pou de tête, responsable de pédiculoses du cuir chevelu et ou pédiculose de la tête.

La pédiculose de la tête est la plus répandue. C'est une affection endémique très fréquente touchant tous les pays et tous les milieux socio-économiques. Elle affecte surtout les enfants entre 5 et 11 ans.

Le pou de tête est un petit insecte de couleur grisâtre qui se nourrit du sang de son hôte trois à cinq fois par jour. Leurs oeufs ou lentes, de forme ovale, se fixent très solidement aux cheveux grâce à la spumaline, matière collante secrétée par les lentes. Chaque lente est généralement collée à un cheveu, la plupart du temps tout près de la peau.

Les poux de tête ne peuvent ni sauter, ni voler, et se transmettent surtout par un contact tête à tête. Ils peuvent être présents pendant plusieurs mois sur le cuir chevelu sans que le porteur ne s'en rende compte, ce qui nuit à la mise en place de mesures prophylactiques adéquates et favorise la transmission. L'infestation est habituellement sans symptôme durant les trois à quatre premières semaines, puis des démangeaisons apparaissent généralement, dues à la présence de substances allergènes dans la salive des parasites.

Dans le traitement des pédiculoses de la tête plusieurs approches peuvent être utilisées. En première intention, il est possible de peigner les cheveux pour éliminer les poux qui meurent rapidement une fois qu'ils sont séparés de leur hôte, mais cette solution ne permet d'éliminer qu'une partie des poux et ne peut aucunement être utilisée seule, sauf pour les enfants en très bas âge. Cette solution nécessite donc de réitérer l'opération de nombreuses fois.

Aussi, l'approche la plus courante consiste à appliquer un insecticide sur le cuir chevelu. Plusieurs traitements ont ainsi été proposés. Les premiers traitements locaux développés sont des produits à base d'insecticides, tels que des organophosphorés comme le malathion, des perméthrines ou des pyréthrines, associés à un agent synergique tel que le butoxyde de pipéronyle. Ces traitements, notamment ceux à base de malathion sont considérés comme étant efficaces. Toutefois, il s'agit de produits très irritants pour le cuir chevelu, qui présentent en outre une odeurforte désagréable et qui sont considérés comme toxiques et polluants pour l'environnement. En outre, il est apparu que leur efficacité diminuait avec le temps du fait d'une adaptation enzymatique des poux, si bien que ce type de produit est délaissé.

Des traitements locaux à base de siloxanes notamment à base de diméticone ont également été développés, comme ceux décrit dans la demande WO-01/19190. Ces produits agissent en noyant les poux dans une phase huileuse, qui meurent par asphyxie. Néanmoins, ces produits ne sont pas suffisamment efficaces en particulier contre les lentes et nécessitent plusieurs applications répétées. En outre, ils rendent les cheveux gras, et sont très inflammables. Par ailleurs, les silicones utilisées ne sont pas biodégradables ce qui est dommageable pour l'environnement. Enfin, des études récentes mettent en évidence que les siloxanes (silicone) sont des perturbateurs endocriniens sévères ce qui plaide pour une limitation voir une interdiction de leur utilisation.

On connaît encore des produits insecticides à base de siloxanes et d'esters d'acides gras, tels que ceux décrits dans la demande EP-1.499.184. Ces produits agissent en dissolvant la chitine qui recouvre l'exosquelette du pou, provoquant sa déshydratation puis sa mort. Ils sont connus sous le nom commercial Resultz® constitués à 50% par de la Cyclométhicone, très inflammable, et à 50% par du myristate d'isopropyle. Néanmoins, leur efficacité sur les poux adultes est très variable et leur pouvoir sur les lentes n'a pas été établi. En outre, ces produits ne peuvent pas être appliqués chez les enfants de moins de 4 ans et leur utilisation est fastidieuse puisqu'elle nécessite une application sur cheveux secs, un temps de pose d'au moins 8 heures et plusieurs shampoings des cheveux et du cuir chevelu pour être éliminé et diminuer le côté gras de la chevelure. Une nouvelle application, voire deux applications supplémentaires sont nécessaires pour éliminer les lentes écloses après un intervalle de 7 jours. Ces produits rendent également les cheveux gras et sont inflammables.

Des formulations de types microémulsion ont également été développées et sont notamment décrites dans la demande FR0955753, cependant ces formulations ne sont totalement efficaces sur les poux qu'après un temps d'application d'une heure, ce qui est long par rapport à la demande actuelle des consommateurs. De plus, l'efficacité sur les lentes n'est pas complète.

Des produits à base de Chlorure de Sodium ont été décrits avec une efficacité satisfaisante par rapport aux standards actuels. Néanmoins, ils laissent, du fait de leur contenu salin, un cheveu rêche et peu coiffable. Le caractère visqueux des formulations est également incompatible avec une forme spray plus facile d'utilisation.

Plus récemment, des formulations à base d'une solution huileuse constituée au moins par une phase dispersante huileuse dans laquelle est dispersée de l'alcool benzylique ont été décrite dans la demande WO2019/053134. Cependant, ces formulations notamment à base de particule de silice peuvent être instables dans leur contenant. En effet, l'interaction des particules de silice avec le contenant et particulièrement les contenants en verre est susceptible d'entraîner une gélification hétérogène de la formulation et de fait une instabilité du produit.

Il existe donc toujours un besoin pour de nouvelles formulations anti-poux, qui soit écoresponsable, rapidement biodégradables, d'origine végétale (y compris non OGM), facile d'utilisation, peu coûteuse, dépourvues d'insecticide toxique et d'huiles de silicone, non inflammable et très efficace à la fois contre les poux de tête et les lentes.

**Résumé de l'invention**

Pour répondre à ce besoin, l'invention propose une composition sous forme d'une solution huileuse pour son utilisation dans la prévention et/ou le traitement des pédiculoses, comprenant entre 30 et 100% d'alcanes liquides, en poids du poids total de la composition, qui soit rapidement biodégradables et d'origine végétale (non OGM). Préférentiellement, l'alcane liquide biodégradable représente entre 30% et 70% en poids de ladite composition, plus préférentiellement entre 40 et 60%.

Selon un objet de la présente invention, la composition peut également comprendre un ou plusieurs agent-tensioactif permettant ainsi de rendre rinçable la composition. Préférentiellement, l'au moins un agent-tensioactif est présent dans la composition à hauteur d'au moins 1%, en poids du poids total de la composition. L'au moins un agent tensioactif présente une balance hydrophile/lipophile inférieure ou égale à 5. Préférentiellement, l'agent tensioactif représente entre 1% et 15% en poids de ladite composition, plus préférentiellement entre 1 et 10%.

Selon un objet de l'invention, l'agent tensioactif est préférentiellement choisi parmi les agents tensioactifs cationiques, anioniques, amphotères et non ioniques. Plus préférentiellement, l'agent tensioactif est le Propylene glycol monolaurate de type II, plus couramment connu sous nom commercial, le Lauroglycol®.

Selon un autre objet de l'invention, la composition peut comprendre au moins deux agents tensioactifs, la combinaison des au moins deux agents tensioactifs présente une balance hydrophile/lypophile globale inférieure ou égale à 5.

Selon un autre objet, la composition peut également comprendre au moins une huile végétale. L'huile végétale représente alors au moins 10% en poids du poids total de la composition. Préférentiellement, l'huile végétale liquide représente entre 10% et 60% en poids de ladite composition.

La présente composition selon l'invention se présente alors préférentiellement sous forme d'une solution huileuse et présente une efficacité pédiculicide et lenticide totale et ce en une seule application d'une durée de moins de 15 minutes, préférentiellement d'une durée inférieure ou égale à 5 minutes. En outre, lorsqu'un ou plusieurs agents tensio-actifs sont présent(s) dans la composition selon l'invention, celle-ci est très facilement rinçable et d'une grande facilité d'utilisation, y compris chez les enfants.

Selon un autre objet, la composition selon l'invention peut également comprendre d'autre ingrédients. Aussi, la composition peut comprendre au moins ingrédient choisi parmi un agent antioxydant, une substance pédiculicide, une substance répulsive contre les poux, un agent conservateur, et leur combinaison.

Lorsque la composition comprend un agent antioxydant, celui-ci représente au plus 1% en poids de la composition. Avantageusement, l'agent antioxydant est choisi parmi le butylhydroxytoluène, le butylhydroxyanisol et le palmitate d'ascorbyle.

Lorsque la composition comprend une substance pédiculicide, celle-ci représente au plus 1% en poids de la composition. Préférentiellement, la substance pédiculicide est choisi parmi le malathion, les perméthrines, des essences végétales ou des huiles naturelles pédiculicides et des inhibiteurs de la croissance des lentes.

Lorsque la composition comprend une substance répulsive, celle-ci représente au plus 1% en poids de la composition. Préférentiellement, la substance répulsive est choisie parmi l'essence de myrte, la citronnelle, l'éthyl butylacetylaminopropionate (IR 3535), le DET (Diéthyltoluamide), les huiles essentielles de citron, de lavande, de géranium rosat, de romarin cinéole, de célerie et de tea-tree.

Lorsque la composition comprend un agent conservateur, celui-ci représente au plus 3% en poids de la composition.

D'autre part, la composition selon l'invention présente une faible viscosité, ce qui permet de les proposer sous forme de lotions fluides ou de spray facile d'application. Selon une variante, il est également possible de gélifier ces compositions, par l'ajout d'un agent gélifiant, ce qui peut est avantageux afin d'éviter que la composition ne coule dans les yeux des sujets traités, en particulier des enfants. Préférentiellement, il représente entre 3 et 10% en poids de la composition. L'agent gélifiant peut être choisi parmi les copolymères de siloxane et les copolymères de polyamides.

L'invention a donc pour objet particulier la composition sous forme d'une solution huileuse éventuellement rinçable pour une utilisation en application topique sur le cuir chevelu et les cheveux dans la prévention et/ou le traitement des pédiculoses de la tête chez l'homme ou selon une variante sur le pelage animal dans la prévention et/ou le traitement des pédiculoses animales.

D'autres caractéristiques et avantages ressortiront de la description détaillée de l'invention et des exemples qui vont suivre.

**Description détaillée de l'invention**

<u>Définition</u>

Par « Alcanes liquides biodégradables d'origine végétale (non OGM) » au sens de l'invention on entend des alcanes 100% issus d'une biomasse végétale et responsable. On peut citer à titre d'exemple, l'huile de palme qui est non OGM, traçable jusqu'aux plantations et couverte par un certificat Mass Balance selon le référentiel RSPO. Ils sont aussi facilement biodégradables, c'est-à-dire entre 86% et 89% de biodégradabilité à 28 jours, selon le protocole OCDE 301B. D'un point de vue chimique, les alcanes sont des dérivés en C12-16 linéaire ou branché ou des alcanes en C17-18 branchés alcanes. A titre d'exemple, on peut citer les alcanes correspondant au CAS 90622-53-0 ou CAS 2081854-12-6. Plus préférentiellement, les alcanes selon l'invention sont commercialisés par la Société SEPPIC sous les noms de marque Emogreen L15 et L19 et c69 sous le numéro CAS 64742-46-7.

Par « rinçable » au sens de l'invention on entend l'action de rincer avec de l'eau tiède du robinet la composition selon l'invention présente sur les cheveux ou la peau sans avoir à ajouter un savon ou un shampoing supplémentaire en plus de la composition.

Par « solution huileuse » au sens de l'invention on entend une solution homogène huileuse. Préférentiellement, celle-ci est obtenue, lorsque la composition comprend plusieurs ingrédients, sous agitation modérée à température ambiante des autre ingrédients de la composition tels que un agent tensioactif et ou une ou des huiles végétales.

Par « balance hydrophile/lipophile » ou « Hydrophilic-Lipophilic Balance » ou « équilibre hydrophile/lipophile » ou « HLB » au sens de l'invention on entend une grandeur caractéristique d'un tensio-actif qui est mesurée par une méthode, proposée en 1949 par Griffin permettant de chiffrer l'équilibre existant entre la partie hydrophile et la partie lipophile de la molécule de tensioactif ; équilibre lié à la solubilité dans l'eau. L'échelle varie de 0 à 20 : plus la valeur est élevée, plus la solubilité dans l'eau est grande. D'après la méthode de Davies, elle correspond à l'équation suivante :

[Math. 1]

$$ HLB = \sum HLB_{groupes\ hydrophiles} - \sum HLB_{groupes\ hydrophobes} + 7 $$

Les tensioactifs sont souvent utilisés en mélange et dans ce cas la valeur HLB d'un mélange binaire se calcule en première approximation par la relation linéaire suivant l'équation de Brochette (Brochette, 1999: Elaboration et étude des émulsions. Techniques de l'ingénieur, traité Génie des procédés 12150 : 1-18 ) avec m1 : masse du tensioactif 1 dans la formulation, m2 : masse du tensioactif 2 dans la formulation, HLB1 : valeur HLB du tensioactif 1, HLB2 : valeur HLB du tensioactif 2.

[Math. 2]

$$ HLB\ m\acute{e}lange = [(m1/m1+m2)\ x\ HLB1] + [(m2/m1+m2)xHLB2] $$

Les valeurs de HLB sont ainsi additives. Aussi par «balance globale hydrophile/lipophile » ou « balance hydrophile/lipophile globale » ou « équilibre hydrophile/lipophile global » ou « HLB global» au sens de l'invention on entend la somme des valeurs HLB des différents agents tensioactifs présents dans la solution.

Composition

La présente invention a donc pour objet une composition sous forme d'une solution huileuse ou éventuellement gélifiée pour son utilisation dans la prévention ou le traitement des pédiculoses, comprenant au moins 30% d'au moins un alcane liquide biodégradable d'origine végétale (non OGM), en poids du poids total de la composition.

Les alcanes sont des ingrédients bien connus de l'industrie cosmétique pour leurs propriétés sensorielles et très utilisés. Historiquement, ils sont d'origine synthétique ou minérale et ne sont donc pas adaptés pour les compositions plus naturelles et respectueuses de l'environnement. Aussi, la présente invention vise uniquement des alcanes d'origine uniquement végétale, certifiés et approuvés notamment par COSMOS et/ou ECOCERT.

Ainsi, les alcanes selon l'invention sont à 100% issus d'une biomasse végétale et responsable. A titre d'exemple, l'huile de palme qui est non OGM, traçable jusqu'aux plantations et couverte par un certificat Mass Balance selon le référentiel RSPO. En outre, les alcanes selon l'invention sont aussi facilement biodégradables, entre 86% et 89% de biodégradabilité à 28 jours, selon le protocole OCDE 301B.

Sur le plan chimique, les alcanes selon l'invention sont des alcanes en C12-16 linéaire ou branché ou des alcanes en C17-18 branchés alcanes. Plus préférentiellement les alcanes selon l'invention sont les alcanes sous le numéro CAS 90622-53-0 ou CAS 2081854-12-6.

De façon particulièrement préféré, les alcanes selon l'invention sont des alcanes C15-19, notamment commercialisés par la Société SEPPIC sous les noms de marque Emogreen L15 et L19 et c69.

Aussi, la composition selon l'invention comprend au moins un alcane liquide biodégradable d'origine végétale (non OGM), ledit alcane est une phase dispersante lipophile qui représente la base de la solution huileuse de la présente composition.

Très préférentiellement, la composition selon l'invention, sous forme d'une solution huileuse ne comprend pas d'eau.

Préférentiellement, l'alcane liquide biodégradable d'origine végétale (non OGM) représente au moins 30% en poids du poids total de la composition selon l'invention, plus préférentiellement entre 30 et 70% en poids du poids total de la composition, encore plus préférentiellement entre 40 et 60% en poids du poids total de la composition.

Avantageusement, entre 50 et 100 % d'alcanes liquides biodégradables d'origine végétale (non OGM), en poids du poids total de la composition permet d'obtenir une très bonne efficacité sur les poux et lentes à cinq minutes après application de la composition selon l'invention.

Le mécanisme d'action des alcanes liquides biodégradables d'origine végétale (non OGM) consiste à obstruer les orifices respiratoires des poux et des lentes dénommés spiracles provoquant ainsi un choc osmotique qui détruit instantanémment le système digestif des parasites provoquant ainsi leur mort immédiate.

La composition selon l'invention peut également comprendre d'autres ingrédients, les ingrédients sont alors tous mélangés sous agitation modérée à température ambiante ou sous une température modérée inférieure à 60°C.

Selon un mode de réalisation préféré, la composition comprend également :

- au moins 1% d'au moins un agent tensioactif ayant une balance hydrophile/lipophile inférieure ou égale à 5, et
- au moins 10 % d'au moins une huile végétale,

les pourcentages étant donnés en poids du poids total de la composition.

Aussi, lorsque les alcanes liquides biodégradables sont associés à un tensio actif présentant une balance hydrophile/lipophile inférieure à 5, la composition selon l'invention est facilement rinçables et laisse les cheveux non gras et soyeux.

La composition sous forme d'une solution huileuse rinçable comprend alors préférentiellement au moins 1% d'au moins un ou plusieurs agents tensioactifs ayant une balance hydrophile/lipophile inférieure ou égale à 5, la composition est facilement rinçable et par conséquent utilisable en une seule étape sans besoin d'application d'un shampoing traditionnel.

De plus, l'agent tensioactif permet de diminuer la tension superficielle du milieu qui facilite la pénétration de la formulation au sein des poux et des lentes contribuant ainsi à renforcer l'efficacité de la composition. L'agent tensioactif présent dans la composition selon l'invention est préférentiellement choisi parmi les agents tensioactifs cationiques, anioniques, amphotères et non ioniques ou leurs mélanges.

Ledit agent tensioactif présent dans la composition représente préférentiellement entre 1% et 15% en poids du poids total de ladite composition, plus préférentiellement entre 1% et 10%. Ceci est particulièrement avantageux pour conférer un caractère particulièrement rinçable à la formulation.

Selon un autre objet de l'invention, la composition peut également comprendre une combinaison d'au moins deux agents tensioactifs dont la balance hydrophile/lypophile de ladite combinaison est inférieure ou égale à 5. La combinaison d'au moins deux agents tensioactifs étant particulièrement adapté pour optimiser, améliorer la pénétration de la formulation au sein des poux et des lentes ainsi que de facilité le rinçage des cheveux. En effet, la nature des cheveux, notamment chez les humains, étant particulièrement hétérogène, la combinaison d'au moins deux agents tensioactifs est particulièrement d'intérêt pour améliorer l'efficacité de rinçage de la solution après application.

A titre d'exemple, on peut citer, ci-après, les agents tensioactifs présentant une balance hydrophile/lipophile (HLB) inférieure ou égale à 5 et permettant d'obtenir une solution miscible avec l'au moins un alcane liquide biodégradable d'origine végétale (non OGM):

- Glycol Distearate HLB = 1
- Sorbitan Trioleate ou Span 85 HLB = 1.8
- Propylene Glycol Isostearate HLB = 2.5
- Glycol Stéarate HLB = 2.9
- Lauroglycol™ 90, Propylene glycol monolaurate (type II) HLB 3
- Sorbitan Sesquioleate HLB = 3.7
- Glyceryl Stéarate HLB = 3.8
- Lecithin HLB = 4
- Sorbitan Oleate ou Span 80 HLB = 4.3
- Sorbitan Monostearate NF HLB = 4.7
- Sorbitan Stéarate HLB = 4.7
- Sorbitan Isostearate HLB = 4.7
- Steareth-2 HLB = 4.9
- Oleth-2 HLB = 4.9
- Lauroglycol™ FCC, Propylene glycol monolaurate (type I) HLB = 5

Très préférentiellement, l'agent tensioactif est le Propylene glycol monolaurate de type II, commercialisé sous le nom commercial Lauroglycol®, ayant également une balance hydrophile/lipophile égale à 5.

La composition peut également comprendre au moins une huile végétale permettant d'améliorer les caractéristiques organoleptiques des préparations en jouant sur leur fragrance, leur viscosité.

A titre d'exemple, les huiles végétales naturelles peuvent être choisie parmi l'huile d'olive, l'huile de colza, l'huile de tournesol, le myristate d'isopropyle (MIP), les huiles issues de graines telles que l'huile de sésame, huile de lin et huile de chanvre ; les huiles issues de fruits à coque telles que l'huile de noix et huile de noisette ; les huiles issues de pépins telles que l'huile de pépins de raisin et huile de pépins de courge ; l'huile d'amande douce, l'huile de coco, l'huile d'argan, l'huile de jojoba, l'huile d'avocat, l'huile de nigelle, l'huile de rose musquée, l'huile de camélia, et leurs combinaisons.

Préférentiellement, l'huile végétale représente au moins 10% en poids du poids total de ladite composition, plus préférentiellement entre 10 et 60%. De manière particulièrement avantageuse, l'huile végétale liquide représente entre 30% et 60% en poids du poids total de la composition.

Selon un objet préféré, l'huile végétale est un mélange d'huile végétale, au moins deux huiles végétale, au moins trois huiles végétales. Lesdites huiles végétales sont préférentiellement choisis parmi les huiles listées citées ci-dessus, et leur mélange.

Selon un objet particulièrement préféré, la composition selon l'invention est constituée exclusivement d'au moins 50% d'alcanes liquides biodégradables d'origine végétale (non OGM), d'au moins 5% d'au moins un agent tensioactif ayant une balance hydrophile/lipophile inférieure ou égale à 5, et d'au moins 45% d' huile végétale liquide, les pourcentages étant donnés en poids du poids total de la composition.

Selon un autre objet, la composition selon l'invention peut également comprendre d'autres ingrédients dispersés dans la solution huileuse, en plus du ou des agents tensioactifs. Il peut s'agir en particulier :

- d'un agent antioxydant, comme par exemple le butylhydroxytoluène, le butyhydroxyanisol ou le palmitate d'ascorbyle, qui permet de prévenir l'oxydation de la phase huileuse et ainsi son rancissement, et/ou

- d'une substance pédiculicide, qui permet d'améliorer l'efficacité de la composition, comme par exemple le malathion, les perméthrines, des essences végétales ou des huiles naturelles pédiculicides tel que l'essence d'anis, l'huile de l'arbre à thé, l'essence de myrte, l'essence de lavande, l'huile de neem et des inhibiteurs de la croissance des lentes tels que le Farnesol et le Nérolidol, et/ou

- d'une substance répulsive contre les poux, comme par exemple l'essence de myrte, la citronnelle, l'éthyl butylacetylaminopropionate (IR 3535), le DET (Diéthyltoluamide), les huiles essentielles de citron, de lavande, de géranium rosat, de romarin cinéole, de céleri et de tea-tree, et/ou

- d'un agent gélifiant, qui peut notamment être choisi parmi les copolymères de siloxane et de polyamides (comme par exemple Oléocraft LP20), et/ou

- d'un agent conservateur, comme par exemple l'acide benzoïque.

Préférentiellement, l'agent antioxydant, lorsqu'il est présent dans la composition selon l'invention, représente au plus 1% en poids de la composition,

Préfentiellement, la substance pédiculicide présent dans la composition, représente au plus 1% en poids de la composition et ce, afin d'éviter des effets indésirables, notamment toxicité, irritation.

Préfentiellement, la substance répulsive présente dans la composition, représente au plus 1% en poids de la composition

Préférentiellement l'agent gélifiant lorsqu'il est présent dans la composition selon l'invention, représente au plus 30% en poids de la composition, encore plus préférentiellement entre 1 et 15%.

Préférentiellement l'agent conservateur lorsqu'il est présent dans la composition selon l'invention, représente au plus 3% en poids de la composition, de façon préférée entre 0,1 et 3%.

La composition selon l'invention présente très préférentiellement une ou plusieurs de ces caractéristiques, de façon préférée toutes : stable, transparente, rinçable à l'eau et très peu voire non irritante, non toxique, très peu voire non inflammable, biodégradable et dépourvues d'effet perturbateurs endocriniens à l'opposé des dérivés de type siloxane ou silicone.

Procédé

La composition selon l'invention peut être obtenue par tout procédé adapté. Il peut s'agir en particulier d'une simple étape de ré-homogénéisation lorque la composition est consistuée exclusivement d'alacane liquide biodégradable. Lorsque la composition comprend plusieurs ingrédients, le procédé peut consister en la mise en oeuvre des étapes suivantes à température ambiante :

- solubilisation du ou des agents tensioactifs dans les Alcanes liquides biodégradables d'origine végétale (non OGM), éventuellement accompagné d'une huile végétale,
- éventuellement ajout d'un antioxydant dans la phase huileuse,
- éventuellement ajout d'un conservateur dans la phase huileuse.

Dans le cas où l'on souhaite gélifier la composition, il est possible d'ajouter les étapes suivantes :

- Chauffer à environ 50°C et disperser le copolymère de polysiloxane et de polyamide,
- Faire refroidir la préparation.

La composition selon l'invention peut être utilisée en particulier sous forme de lotion ou sous forme de spray, ces deux formes étant particulièrement faciles d'utilisation pour des applications sur les cheveux et le cuir chevelu. Selon une variante, la composition, lorsqu'elle comprend un agent gélifiant, peut également se présenter sous forme de gel.

La composition est particulièrement d'intérêt comme produit de prévention et/ou de lutte contre les poux de tête et/ou les contre les lentes, notamment pour être appliquée sur les cheveux et/ou le cuir chevelu ou sur le pelage d'animaux comme les chevaux.

La composition selon l'invention peut être appliquée indifférament sur cheveux secs ou humides.

Un mode d'application particulier consiste à imprégner la chevelure sèche avec la composition selon l'invention et à rincer la chevelure avec un shampooing pour les formulations dépourvues d'agents tensio-actifs soit directement avec de l'eau tiède lorsqu'elles renferment un agent tensio-actif entre deux minutes et une heure après l'application.

Avantageusement, il n'est pas nécessaire de répéter l'application après 7 jours étant donné la remarquable efficacité de la composition sur les lentes.

Selon un autre avantage, la composition selon l'invention laisse les cheveux soyeux, peignables et esthétiquement parfaits, contrairement aux produits de l'art antérieur qui laissent les cheveux gras, collants, rêches après application.

De plus, la composition selon l'invention présente un pouvoir de dissolution important notamment vis-à-vis de la spumaline, colle naturelle et forte sécrétée par les lentes qui rend difficile leur décrochage. Ainsi l'application des formulations permet une élimination facilitée des lentes.

L'invention est à présent illustrée par des exemples de compositions selon l'invention et par des essais d'efficacité pédiculicide et lenticide en particulier des essais comparatifs par rapport au produit commercialisé sous l'appelation POUXIT® Classic.

**Exemples**

Exemple 1 : Formulation liquide

La formulation de la solution huileuse selon l'exemple 1 est présentée dans le Tableau 1.

[Tableau 1]

| Ingrédients | % |
|---|---|
| Alcanes liquides biodégradables d'origine végétale - EMOGREEN™ L19 | 100 |
| TOTAL | 100 |

La formulation se présente alors sous la forme d'une lotion facile d'application sur les cheveux.

Exemple 2 : Formulation liquide

La formulation de la solution huileuse selon l'exemple 2 est présentée dans le Tableau 2.

[Tableau 2]

| Ingrédients | % |
|---|---|
| Alcanes liquides biodégradables d'origine végétale - EMOGREEN™ L19 | 50 |
| Lauroglycol™ FCC, Propylene glycol monolaurate | 5 |
| Huiles de lin | 48 |
| TOTAL | 100 |

Le procédé de fabrication est décrit ci-après. Sous agitation à température ambiante on mélange les Alcanes liquides biodégradables d'origine végétale (non OGM), les huiles végétales liquide et le Lauroglycol® (Lauroglycol™ FCC, Propylene glycol monolaurate). La formulation se présente sous la forme d'une lotion transparente facile d'application sur les cheveux sous forme de lotion ou de spray et rinçable.

Exemple 3 : Formulation gélifiée

La formulation de la solution gélifiée de l'exemple 3 est présentée dans le Tableau 3.

[Tableau 3]

| Ingrédients | % |
|---|---|
| Alcanes liquides biodégradables d'origine végétale - EMOGREEN™ L15 | 40 |
| Lauroglycol™ FCC, Propylene glycol monolaurate | 5 |
| MIP | 50 |
| SP Oleocraft LP-20 | 5 |
| | 100 |

Le procédé de fabrication est décrit ci-après. Sous agitation à température ambiante on mélange les Alcanes liquides biodégradables d'origine végétale (non OGM) EMOGREEN™ L19 au Lauroglycol®, au MIP. On chauffe ensuite à 50C° et on ajoute l'Oléocraft® sous agitation vigoureuse. On laisse refroidir lentement. La formulation se présente sous la forme d'une solution gélifiée.

Exemple 4 : Formulation liquide

La formulation de la solution huileuse de l'exemple 4 est présentée dans le Tableau 4.

[Tableau 4]

| Ingrédients | % |
|---|---|
| Alcanes liquides biodégradables d'origine végétale - EMOGREEN™ L15 | 40 |
| Span 80® (Polysorbate 80) | 10 |
| Huile de tournesol | 50 |
| | 100 |

Le procédé de fabrication est décrit ci-après. Sous agitation à température ambiante on mélange les Alcanes liquides biodégradables d'origine végétale (non OGM) EMOGREEN™ L15, le Span 80® et l' huile de tournesol. La formulation se présente sous la forme d'une solution transparente facile d'application en lotion ou en spray sur les cheveux et rinçable.

Exemple 5 : Formulation gélifiée

La formulation de la solution gélifiée de l'exemple 5 est présentée dans le Tableau 5

[Tableau 5]

| Ingrédients | % |
|---|---|
| Alcanes liquides biodégradables d'origine végétale (non OGM) EMOGREEN™ L15 | 83,5 |
| Span 80® | 7,5 |
| Huiles de lin | 4 |
| SP Oleocraft LP-20 | 5 |
| | 100 |

Le procédé de fabrication est décrit ci-après. Sous agitation à température ambiante on mélange les Alcanes liquides biodégradables d'origine végétale (non OGM) EMOGREEN™ L15, l' huile de lin, le Span80®. Le mélange est chauffé à 50C° sous agitation vigoureuse et l'Oléocraft® LP 20 est introduit. On laisse le mélange refroidrir à température ambiante sous agitation. La formulation se présente sous la forme d'une solution gélifiée.

Exemple 6 : Formulation liquide

La formulation de la solution huileuse de l'exemple 6 est présentée dans le Tableau 6.

[Tableau 6]

| Ingrédients | % |
|---|---|
| Alcanes liquides biodégradables d'origine végétale - EMOGREEN™ L15 | 40 |
| Span 85® | 15 |
| MIP | 45 |
| | 100 |

Le procédé de fabrication est décrit ci-après. Sous agitation à température ambiante on mélange les Alcanes liquides biodégradables d'origine végétale (non OGM) EMOGREEN™ L15, le Span 85® et le MIP. La formulation se présente sous la forme d'une solution transparente facile d'application en lotion ou en spray sur les cheveux et rinçable.
Ainsi, les formulations selon les exemples 1 à 6 sont particulièrement adaptées à l'utilisation revendiquée.

**Essais d'efficacité de la composition selon l'invention en comparaison avec une autre composition.**

Des essais d'efficacité pédiculicide et lenticide de compositions selon les différents exemples 1 à 6 en comparaison avec une formulation huileuse à base de diméthicone commercialisée sous le nom de POUXIT® Classic et avec un contrôle sont décrits ci-après.

Les activités pédiculicides et ovicides de POUXIT® Classic et des exemples 1 à 6 ont été testés en laboratoire sur le pou du corps humain selon le mode opératoire suivant.

Les poux (*Pediculus humanus humanus*) sont élevés en laboratoire selon la méthode décrite par Cole (1966). Pour chaque essai, 50 poux (10 mâles, 10 femelles et 30 nymphes) ont été placés sur un disque de papier filtre blanc de 7 cm et exposés à 1g de la préparation à l'essai. Les poux sont restés en contact avec la substance pendant 5 minutes pour les formulations décrites dans la présente invention et pendant une heure pour POUXIT CLASSIC selon le mode opératoire préconisé par le laboratoire exploitant. Ils ont été retirés du papier filtre, lavés avec un shampooing et rincés à l'eau du robinet pendant 1 minute. Après le traitement, les poux ont été transférés sur un disque de papier filtre frais et incubés toute la nuit à une température et une humidité optimales. La mortalité a été déterminée après 24 heures.

Les poux ont été placés sur des cheveux humains tous les deux jours pendant 5 jours et laissés pendant 24-48 heures pour la ponte. Cinquante oeufs âgés de 2 à 6 jours ont été traités de la même façon que les poux. Le nombre d'oeufs éclos et non éclos a été compté après 10 jours.

Comme contrôle, on a utilisé des poux traités avec de l'alcool éthylique à 40 %. Chaque temps d'exposition a été testé en trois exemplaires. Les résultats sont exprimés en pourcentage de poux morts.

Les compositions testées sont présentées dans le Tableau 7.

[Tableau 7]

| COMPOSITION | Temps de contact (minute) | Mortalité chez les poux (moyenne exprimée en %) | Mortalité chez les lentes (moyenne exprimée en %) |
|---|---|---|---|
| POUXIT® CLASSIC | 60 | 65 | 52 |
| (Exemple 1) - Liquide non rinçable 100% alcanes | 5 | 100 | 100 |
| (Exemple 2) - Liquide rinçable - 50% alcanes | 5 | 95 | 89 |
| (Exemple 3) - Gel rinçable 40% alcanes | 5 | 89 | 88 |
| (Exemple 4) - Liquide rinçable 40% alcanes | 5 | 90 | 89 |
| (Exemple 5) - Gel rinçable 83,5% alcanes | 5 | 100 | 98 |
| (Exemple 6) - Liquide rinçable 40% alcanes | 5 | 89 | 100 |
| Contrôle | 60 | 8.0 | 12.0 |

De façon suprenante, les inventeurs ont constaté avec la composition selon l'exemple 1 que les Alcanes liquides biodégradables d'origine végétale (non OGM) EMOGREEN™ L19 seuls présentent une activité pédiculicide totale en après 5 minutes de contact avec les poux et surtout les lentes ce qui n'a à notre connaissance jamais été décrit précédemment.

Ces résultats sont à comparer avec les faibles performances du produit de référence POUXIT® Classic à base de diméthicone .

Pour ce dernier après une heure de contact au lieu de cinq minutes pour les formulations décrites dans cette invention seulement 65% des poux et 52% des lentes sont mortes.

Pour l'ensemble des compositions selon l'invention, on constate une parfaite efficacité pédiculicide et surtout lenticide avec seulement 5 minutes de contact ce qui est remarquable et jamais décrit pour des produits dépourvu d'insecticide et réalisé à base d'Alcanes liquides biodégradables d'origine végétale (non OGM) de type EMOGREEN.

Ces nouvelles compositions selon l'invention à base d'Alcanes liquides biodégradables d'origine végétale (non OGM) de type EMOGREEN permet une meilleure efficacité, 5 minutes au lieu d'une heure pour la plupart des produits concurrents, notamment POUXIT® Classic. De plus, elles présentent une supériorité sur le plan organoleptique, elles sont rinçables lorsqu'un agent tensioactif est ajouté et permettent ainsi de réaliser le traitement en une seule étape en s'affranchissant d'un shampooing.

Aussi, les compositions selon l'invention permettent de surmonter tous les inconvénients de l'art antérieur et de répondre au problème technique de la présente invention.

## Revendications

1. Composition sous forme d'une solution huileuse pour son utilisation dans la prévention ou le traitement des pédiculoses, comprenant au moins 30% d'au moins un alcane liquide, en poids du poids total de la composition.

2. Composition pour son utilisation selon la revendication précédente, **caractérisée en ce que** l'alcane est un alcane d'origine naturelle biodégradable non OGM.

3. Composition pour son utilisation selon la revendication précédente, **caractérisée en ce que** la composition comprend également :

   - au moins 1% d'au moins un agent tensioactif ayant une balance hydrophile/lipophile inférieure ou égale à 5, et
   - au moins 10% d'au moins une huile végétale, les pourcentages étant donnés en poids du poids total de la composition.

4. Composition pour son utilisation selon l'une des revendications 2 ou 3, **caractérisée en ce que** :

   - l'alcane liquide biodégradable représente entre 30% et 70% en poids de ladite composition, et/ou
   - l'agent tensioactif représente entre 1% et 15% en poids de ladite composition, et/ou
   - l'huile végétale liquide représente entre 10% et 60% en poids de ladite composition.

5. Composition pour son utilisation selon l'une des revendications précédentes, **caractérisée en ce que** l'agent tensioactif est choisi parmi les agents tensioactifs cationiques, anioniques, amphotères et non ioniques.

6. Composition pour son utilisation selon l'une des revendications précédentes, **caractérisée en ce que** l'agent tensioactif est le Propylene glycol monolaurate (type II).

7. Composition pour son utilisation selon l'une des revendications précédentes, **caractérisée en ce que** la composition comprend au moins deux agents tensioactifs dont la balance hydrophile/lypophile globale est inférieure ou égale à 5.

8. Composition pour son utilisation selon l'une des revendications précédentes, **caractérisée en ce que** la composition comprend également :

   - au moins un agent antioxydant, et/ou
   - au moins une substance pédiculicide, et/ou
   - au moins une substance répsulsive contre les poux, et/ou
   - un agent conservateur, et/ou
   - un agent gélifiant.

9. Composition pour son utilisation selon la revendication précédente, **caractérisée** en que l'agent antioxydant représente au plus 1% en poids de la composition et/ou l'agent antioxydant est choisi parmi le butylhydroxytoluène, le butylhydroxyanisol et le palmitate d'ascorbyle.

10. Composition pour son utilisation selon l'une des revendications 8 ou 9, **caractérisée** en que la substance pédiculicide représente au plus 1% en poids de la composition et/ou la substance pédiculicide est choisi parmi le malathion, les perméthrines, des essences végétales ou des huiles naturelles pédiculicides et des inhibiteurs de la croissance des lentes.

11. Composition pour son utilisation selon l'une des revendications 8 à 10, **caractérisée** en que la substance répulsive représente au plus 1% en poids de la composition et/ou la substance répulsive est choisi parmi l'essence de myrte, la citronnelle, l'éthyl butylacetylaminopropionate (IR 3535), le DET (Diéthyltoluamide), les huiles essentielles de citron, de lavande, de géranium rosat, de romarin cinéole, de célerie et de tea-tree.

**12.** Composition pour son utilisation selon l'une des revendications 8 à 11, **caractérisée en ce que** l'agent conservateur représente au plus 3% en poids de la composition.

**13.** Composition pour son utilisation selon l'une des revendications précédentes, **caractérisée en ce que** la composition se présente sous forme de lotion ou de spray.

**14.** Composition pour son utilisation selon l'une des revendications 8 à 12, **caractérisée en ce que** l'agent gélifiant est choisi parmi les copolymères de siloxane et les copolymères de polyamides et/ou l'agent gélifiant représente entre 3 et 10% en poids de la composition.

**15.** Composition pour son utilisation selon l'une des quelconques revendications précédentes, **caractérisé en ce qu'**elle est sous une forme adaptée à une application topique sur le cuir chevelu et les cheveux dans la prévention ou le traitement des pédiculoses de la tête chez l'homme ou sur le pelage animal dans la prévention ou le traitement des pédiculoses animals.

Europäisches Patentamt

European Patent Office

Office européen des brevets

# RAPPORT DE RECHERCHE EUROPEENNE

Numéro de la demande

EP 23 17 0542

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X | JP 2019 189533 A (OSAKA SEIYAKU KK) 31 octobre 2019 (2019-10-31) * abrégé * * exemples 1-4 * ----- | 1-15 | INV. A61K31/01 A61K9/00 A61K45/06 A61P33/14 |
| X | US 2015/164066 A1 (HARDING RONALD [AU] ET AL) 18 juin 2015 (2015-06-18) * abrégé * * alinéa [0006] – alinéa [0013] * * exemples 1-9 * * revendications 1-26 * ----- | 1-15 | A01N27/00 A01P7/04 A61Q17/02 |
| A | FR 3 103 105 A1 (DERMOWAVE [PT]) 21 mai 2021 (2021-05-21) * exemples 1-6 * * alinéa [0001] * * alinéa [0017] * * revendications 1-24 * ----- | 1-15 | |
| A | AL-QURAISHY SALEH ET AL: "Head louse control by suffocation due to blocking their oxygen uptake", PARASITOLOGY RESEARCH, SPRINGER BERLIN HEIDELBERG, BERLIN/HEIDELBERG, vol. 114, no. 8, 21 mai 2015 (2015-05-21), pages 3105-3110, XP035521214, ISSN: 0932-0113, DOI: 10.1007/S00436-015-4528-6 [extrait le 2015-05-21] * le document en entier * ----- | 1-15 | DOMAINES TECHNIQUES RECHERCHES (IPC) A61K |
| A | BURGESS I F: "HUMAN LICE AND THEIR MANAGEMENT", ADVANCES IN PARASITOLOGY, ACADEMIC PRESS, LONDON, GB, vol. 36, 1 janvier 1995 (1995-01-01), pages 271-342, XP009016560, ISSN: 0065-308X * le document en entier * ----- | 1-15 | |

Le présent rapport a été établi pour toutes les revendications

| Lieu de la recherche | Date d'achèvement de la recherche | Examinateur |
|---|---|---|
| La Haye | 18 août 2023 | Taylor, Mark |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
................................................................
& : membre de la même famille, document correspondant

EPO FORM 1503 03.82 (P04C02)

## ANNEXE AU RAPPORT DE RECHERCHE EUROPEENNE
## RELATIF A LA DEMANDE DE BREVET EUROPEEN NO.

**EP 23 17 0542**

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

**18-08-2023**

| Document brevet cité au rapport de recherche | Date de publication | Membre(s) de la famille de brevet(s) | | Date de publication |
|---|---|---|---|---|
| JP 2019189533 A | 31-10-2019 | JP | 7140364 B2 | 21-09-2022 |
| | | JP | 2019189533 A | 31-10-2019 |
| US 2015164066 A1 | 18-06-2015 | AU | 2014377875 A1 | 07-07-2016 |
| | | CA | 2933081 A1 | 23-07-2015 |
| | | EA | 201691033 A1 | 31-10-2016 |
| | | EP | 3082424 A2 | 26-10-2016 |
| | | JP | 6711751 B2 | 17-06-2020 |
| | | JP | 2017500342 A | 05-01-2017 |
| | | NZ | 720995 A | 27-05-2022 |
| | | US | 2015164066 A1 | 18-06-2015 |
| | | US | 2019216086 A1 | 18-07-2019 |
| | | WO | 2015107384 A2 | 23-07-2015 |
| FR 3103105 A1 | 21-05-2021 | EP | 4058009 A1 | 21-09-2022 |
| | | FR | 3103105 A1 | 21-05-2021 |
| | | WO | 2021094561 A1 | 20-05-2021 |

EPO FORM P0460

Pour tout renseignement concernant cette annexe : voir Journal Officiel de l'Office européen des brevets, No.12/82

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 0119190 A
- EP 1499184 A
- FR 0955753
- WO 2019053134 A

**Littérature non-brevet citée dans la description**

- *CHEMICAL ABSTRACTS,* 90622-53-0
- *CHEMICAL ABSTRACTS,* 2081854-12-6
- *CHEMICAL ABSTRACTS,* 64742-46-7
- **BROCHETTE.** Elaboration et étude des émulsions. *Techniques de l'ingénieur, traité Génie des procédés,* 1999, vol. 12150, 1-18